# FASCICULE DE BREVET EUROPEEN

(11) **EP 1 223 989 B1**
(45) Date de publication et mention de la délivrance du brevet: **26.11.2003**
(21) Numéro de dépôt: 00918949.9
(22) Date de dépôt: 12.04.2000
(51) Int. Cl.: A61L 2/10, A47L 23/22

(54) **DISPOSITIF POUR DESINFECTER LES SEMELLES DE CHAUSSURES D'UN UTILISATEUR**
VORRICHTUNG ZUM DESINFIZIEREN DER SCHUHSOHLEN EINES BENUTZERS
DEVICE FOR DISINFECTING THE SOLES OF A USER'S SHOES

(30) Priorité: 16.04.1999 FR 9904795
(43) Date de publication de la demande: 24.07.2002
(73) Titulaire: Bonduelle, Claude, 75007 Paris (FR)
(72) Inventeur: Bonduelle, Claude, 75007 Paris (FR)
(74) Mandataire: Burbaud, Eric
(86) Numéro de dépôt international: FR0000935
(87) Numéro de publication internationale: WO00062823

(56) Documents cités:
- EP-A- 0 060 148
- EP-A- 0 792 650
- US-A- 4 981 651
- PATENT ABSTRACTS OF JAPAN vol. 015, no. 289 (C-0852), 23 juillet 1991 (1991-07-23) & JP 03 103262 A (MISAWA HOMES CO LTD), 30 avril 1991 (1991-04-30)
- DATABASE WPI Section Ch, Week 199919 Derwent Publications Ltd., London, GB; Class D22, AN 1999-222933 XP002122659 & JP 11 056980 A (TAKAHASHI WORKS KK), 2 mars 1999 (1999-03-02)

## Description

La présente invention est relative aux dispositifs pour désinfecter les semelles de chaussures d'un utilisateur, ces dispositifs constituant notamment des seuils de porte germicides qui forment une barrière antiseptique empêchant la dissémination de micro-organismes nuisibles par transport sous les semelles de chaussures.

Plus particulièrement, l'invention concerne un dispositif pour désinfecter les semelles de chaussures d'un utilisateur, ce dispositif étant destiné à être disposé sensiblement au niveau du sol (intégré ou non dans le sol) et comportant une surface d'appui supérieure sur laquelle l'utilisateur peut poser ses pieds (simultanément ou successivement) pour désinfecter ses semelles de chaussures. De tels dispositifs sont utiles soit dans des applications professionnelles, par exemple pour accéder à des zones stériles ou quitter des zones contaminées en milieu médical ou industriel, soit dans des applications destinées au grand public, pour préserver l'hygiène des habitations, crèches, lieux publics, etc.

Le document EP-A-0 060 148 décrit un exemple d'un tel dispositif de désinfection, consistant en un tapis de sol imbibé de produit liquide antiseptique qui se dépose sous les semelles des chaussures de l'utilisateur lorsque cet utilisateur marche sur le tapis.

Ce dispositif de désinfection de l'art antérieur présente notamment les inconvénients suivants :
- le produit liquide antiseptique peut s'évaporer rapidement, même en dehors de toute utilisation,
- il est difficile de s'assurer de la présence d'une quantité suffisante de produit liquide antiseptique dans le tapis de sol,
- le produit liquide antiseptique peut générer une certaine pollution ou tout au moins créer une odeur désagréable dans la pièce où est installé le tapis de sol,
- et dans la mesure où le produit liquide antiseptique humidifie les semelles des chaussures de l'utilisateur, il peut conduire à ce que l'utilisateur salisse ensuite le sol s'il avait au départ de la terre ou des poussières sous ses chaussures.

La présente invention a notamment pour but de pallier ces inconvénients.

A cet effet, selon l'invention, un dispositif de désinfection du genre en question est essentiellement caractérisé en ce que la surface d'appui supérieure comporte une paroi résistante laissant passer les rayons lumineux ultraviolets, cette paroi résistante étant adaptée pour supporter le poids de l'utilisateur et recouvrant des moyens d'émission adaptés pour émettre des rayons ultraviolets de nature et de quantité acceptables pour la santé humaine, des moyens de commande électriques étant prévus pour faire fonctionner ces moyens d'émission.

Grâce à ces dispositions, on assure une désinfection efficace et rapide des semelles de chaussures de l'utilisateur sans avoir recours à des produits chimiques qu'il est nécessaire de recharger fréquemment, et sans humidifier les semelles de chaussures ni créer de mauvaises odeurs ou de pollution.

Dans des modes de réalisation préférés de l'invention, on peut éventuellement avoir recours en outre à l'une et/ou à l'autre des dispositions suivantes :
- les moyens d'émission sont adaptés pour émettre des rayons ultraviolets ayant des longueurs d'onde comprises au moins dans la plage allant de 200 à 300 nm ;
- les moyens d'émission comprennent au moins une lampe, disposée dans une cavité recouverte par ladite paroi résistante ;
- la lampe émettrice de rayons ultraviolets est une lampe au mercure adaptée pour émettre majoritairement des rayons lumineux ayant une longueur d'ondes voisine de 254 nm ;
- le dispositif comporte en outre des moyens de réflexion des rayons ultraviolets, disposés sous chaque lampe ;
- les moyens de commande comprennent, d'une part, des moyens de déclenchement pour faire débuter l'émission des rayonnements germicides, et d'autre part, des moyens de temporisation pour faire fonctionner les moyens d'émission pendant une durée prédéterminée à partir de l'actionnement des moyens de déclenchement ;
- la durée prédéterminée est comprise entre 1 et 10 s ;
- les moyens de commande comportent des moyens de sécurité nécessitant une clé d'accès pour faire fonctionner les moyens d'émission ;
- les moyens de commande comportent au moins un capteur de présence adapté pour vérifier la présence d'un utilisateur posant au moins un pied sur la surface d'appui supérieure du dispositif de désinfection, et pour interdire l'émission des rayons ultraviolets tant que cette présence n'est pas détectée ;
- des moyens d'occultation sont adaptés pour opacifier la surface d'appui supérieure au moins autour des chaussures de l'utilisateur, en empêchant l'essentiel des rayonnements germicides de se propager vers le haut au-delà desdites chaussures ;
- les moyens d'occultation comprennent au moins une paroi souple qui recouvre la surface d'appui supérieure et au moins partiellement les chaussures de l'utilisateur.

D'autres caractéristiques et avantages de l'invention apparaîtront au cours de la description suivante de deux de ses formes de réalisation, données à titre d'exemples non limitatifs, en regard des dessins joints.

Sur les dessins :
- la figure 1 est une vue schématique en perspective d'un dispositif de désinfection selon une première forme de réalisation de l'invention,
- la figure 2 est une vue en coupe verticale du dispositif de désinfection de la figure 1,
- la figure 3 est un schéma bloc montrant le circuit électrique de commande du dispositif de désinfection des figures 1 et 2,
- et la figure 4 est une vue schématique d'un dispositif de désinfection selon une deuxième forme de réalisation de l'invention.

Sur les différentes figures, les mêmes références désignent des éléments identiques ou similaires.

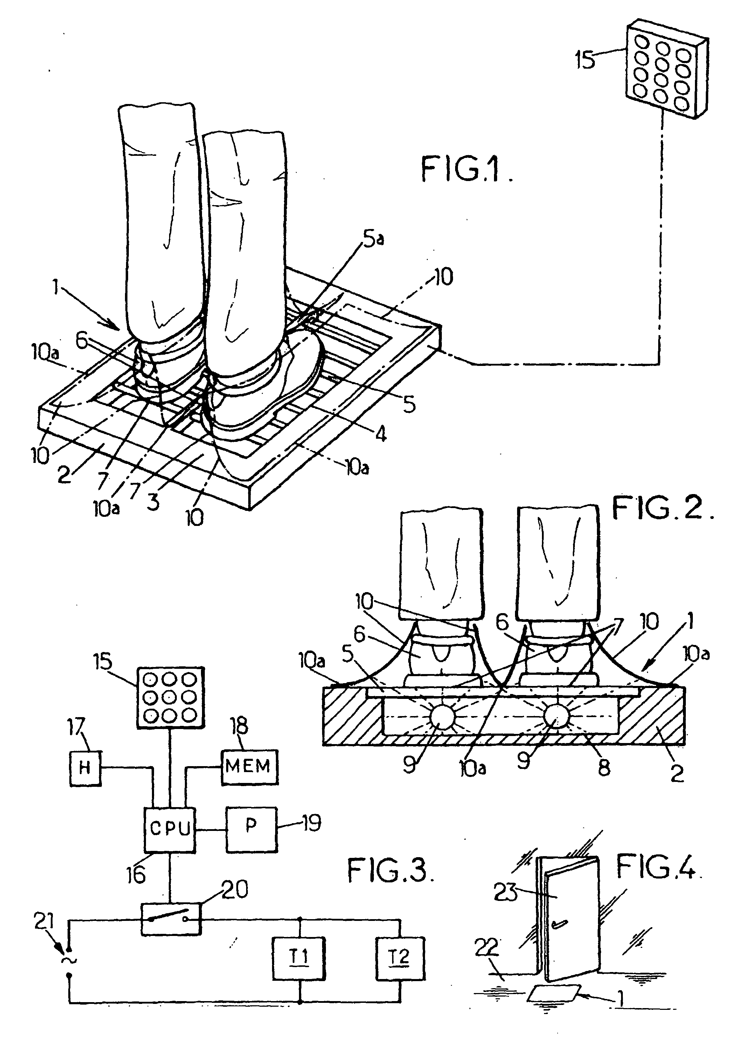

Comme représenté sur les figure 1 et 2, le dispositif de désinfection 1 selon l'invention se présente sous la forme d'un boîtier rigide 2 qui est destiné à être disposé au sol, notamment pour former un seuil de porte germicide, ce boîtier pouvant être soit fixe, soit portatif.

Comme représenté sur la figure 4, le dispositif de désinfection peut le cas échéant être intégré dans le sol 22 à l'intérieur d'une habitation ou d'un local professionnel, au seuil d'une porte 23.

Le boîtier 2 comporte une surface d'appui supérieure 3 (figures 1 et 2) sur laquelle un utilisateur peut se tenir debout. Cette surface d'appui supérieure présente une périphérie opaque qui délimite intérieurement un évidement 4 occupé par une paroi résistante 5 adaptée pour supporter le poids de l'utilisateur et pour laisser passer les rayons lumineux ultraviolets.

Dans l'exemple représenté, cette paroi résistante 5 est constituée par une grille métallique comportant des barreaux relativement fins pour interférer le moins possible avec la transmission des rayons lumineux ultraviolets. Mais ladite paroi résistante pourrait également être constituée par une paroi pleine réalisée en un matériau laissant passer les rayons ultraviolets, par exemple un verre ou un matériau synthétique spécialement traité à cet effet.

Pour désinfecter les semelles 7 de ses chaussures 6, l'utilisateur se place debout sur la grille 5, au-dessus d'une cavité 8 qui contient des lampes 9 émettrices de rayons ultraviolets. A titre d'exemple, la cavité 8 peut comporter deux lampes 9 émettrices de rayons ultraviolets qui peuvent se présenter sous forme de tubes disposés chacun sous l'emplacement d'une des chaussures de l'utilisateur, parallèlement à la longueur de cette chaussure.

Les lampes 9 peuvent être notamment des lampes au mercure basse pression émettant majoritairement des rayons lumineux de type "UV C" dont la longueur d'onde est voisine de 254 nm (à titre d'exemple, de telles lampes, présentées sous forme de tubes, sont commercialisés par la société Philips sous l'appellation "TUV" et par la société OSRAM sous l'appellation "PURITEC"). Plus généralement, les lampes 9 devront de préférence émettre des rayons ultraviolets compris majoritairement dans une zone spectrale de longueurs d'ondes allant de 200 à 300 nm, qui est la zone spectrale d'efficacité maximum des ultraviolets contre les micro-organismes.

Par ailleurs, le dispositif de désinfection 1 comporte des moyens d'occultation adaptés pour empêcher la propagation des rayons ultraviolets vers le haut au-delà des chaussures de l'utilisateur. Dans l'exemple représenté, ces moyens d'occultation sont constitués par des rabats souples 10, par exemple quatre feuilles d'élastomère, savoir :
- deux feuilles extérieures 10 qui sont fixées chacune à la surface supérieure 3 du boîtier par l'intermédiaire de leur bord longitudinal extérieur 10a,
- et deux feuilles intérieures 10 qui sont chacune fixées à une traverse centrale 5a par leur bord longitudinal intérieur 10a.

Les feuilles d'élastomère 10 définissent ainsi deux passages longitudinaux dans lesquels l'utilisateur peut glisser ses chaussures 6 parallèlement aux bords 10a desdites feuilles d'élastomère, les pieds 6 de l'utilisateur entrant dans ces passages par un côté du dispositif 1 et ressortant desdits passages par le côté opposé ou par le même côté après désinfection des semelles.

Comme représenté sur la figure 3, les lampes 9 peuvent être commandées par circuit électronique de commande qui comporte par exemple un clavier 15, une unité centrale électronique telle qu'un microcontrôleur ou microprocesseur 16 (CPU), une horloge 17 (H), une mémoire 18 (MEM) et un capteur de contrainte 19 (P) adapté pour détecter la présence de l'utilisateur sur la grille 5 en mesurant le poids appliqué à cette grille (bien entendu, d'autres capteurs de présence de l'utilisateur seraient utilisables).

L'unité centrale électronique 16 est reliée au clavier 15, à l'horloge 17, à la mémoire 18 et au capteur de présence 19, ainsi qu'à un relais 20 ou autre dispositif de commutation qui commande l'alimentation électrique de deux circuit T1, T2 comprenant chacun l'une des lampes 9.

Les lampes 9 sont alimentées à partir d'une source électrique 21 qui peut être :
- soit le réseau alternatif,
- soit, si l'on souhaite que le dispositif soit aisément déplaçable, une source autonome telle qu'une batterie associée le cas échéant à des moyens de génération de courant alternatif.

Ainsi, lorsque l'utilisateur est en place sur le dispositif de désinfection, il tape un code sur le clavier 15, lequel code est comparé par l'unité centrale 16 à un code prédéterminé contenu dans la mémoire 18 : s'il y a correspondance entre les deux codes, l'unité centrale vérifie, au moyen du capteur de présence 19, que l'utilisateur est positionné sur la grille 5.

Cette vérification faite, l'unité centrale 16 déclenche la fermeture du relais 20, de sorte que les lampes 9 émettent des rayons ultraviolets vers les semelles 7 des chaussures de l'utilisateur.

Après une durée prédéterminée, comprise par exemple entre 1 et 10 s et généralement inférieure à 5 s, l'unité centrale 16 ouvre à nouveau le relais 20, ce qui arrête le fonctionnement des lampes 9. A ce stade, on peut éventuellement prévoir que le dispositif émette un signal sonore à destination de l'utilisateur, pour lui indiquer la fin de la désinfection de ses chaussures.

La durée de désinfection est déterminée à l'avance en fonction de la puissance des lampes 9 et de la géométrie de la cavité 8 et de la grille 5, de façon que les semelles 7 reçoivent une quantité suffisante de rayons ultraviolets pour être un correctement désinfectées. A titre d'exemple, la durée d'émission des rayons ultraviolets pourra être choisie de façon à que les semelles 7 reçoivent une énergie lumineuse au moins égale à 200 J/m² dans la bande spectrale comprise entre 200 et 300 nm, ce qui permet une désinfection efficace contre la majorité des micro-organismes.

Pour maximiser l'énergie reçue par les semelles 7 des chaussures, on peut avantageusement prévoir que le fond de la cavité 8 soit réfléchissant, ou que des réflecteurs soient disposés sous chaque lampe 9.

On notera que, pour simplifier l'utilisation du dispositif de désinfection, le clavier 15 pourrait éventuellement être remplacé par un dispositif de sécurité à clé mécanique ou électronique, ou simplement par un interrupteur disposé hors de portée des enfants.

Bien entendu, le circuit de commande des lampes 9 pourrait être encore plus simplifié, et comporter par exemple uniquement un bouton de commande associé à une temporisation qui fasse fonctionner les lampes 9 pendant une durée prédéterminée à chaque appui sur ledit bouton de commande.

Dans certains cas, les lampes 9 pourraient même fonctionner en permanence.

Bien entendu, et comme il résulte déjà de ce qui précède, la présente invention n'est pas limitée aux exemples de réalisation particuliers qui viennent d'être décrits ; elle en embrasse également les variantes dans lesquelles :
- le dispositif de désinfection aurait une surface supérieure inclinée sur laquelle l'utilisateur poserait successivement ses deux pieds afin de désinfecter ses semelles de chaussures,
- celles dans lesquelles la grille 5 du dispositif de désinfection serait remplacée par une paroi transparente aux ultraviolets dont chaque point peut être opacifié à la demande au moyen d'un système de commande électronique (il peut s'agir par exemple d'une plaque contenant des cristaux liquides), les moyens de commande électroniques en question étant reliés à un système de détection optoélectronique ou autre permettant de détecter l'emplacement exact de chaque semelle de chaussure sur la plaque transparente, afin d'opacifier uniquement les zones de la plaque transparente située autour des semelles de chaussures.

## Revendications

1. Dispositif pour désinfecter les semelles (7) de chaussures d'un utilisateur, ce dispositif étant destiné à être disposé sensiblement au niveau du sol et comportant une surface d'appui supérieure (3) sur laquelle l'utilisateur peut poser ses pieds pour désinfecter ses semelles de chaussures,
**caractérisé en ce que** la surface d'appui supérieure (3) comporte une paroi résistante (5) laissant passer les rayons lumineux ultraviolets, cette paroi résistante étant adaptée pour supporter le poids de l'utilisateur et recouvrant une cavité (8) dans laquelle sont disposés des moyens d'émission (9) adaptés pour émettre des rayons ultraviolets de nature et de quantité acceptables pour la santé humaine, des moyens de commande électriques (15-20) étant prévus pour faire fonctionner ces moyens d'émission (9).

2. Dispositif de désinfection selon la revendication 1, dans lequel les moyens d'émission (9) sont adaptés pour émettre des rayons ultraviolets ayant des longueurs d'onde comprises au moins dans la plage allant de 200 à 300 nm.

3. Dispositif de désinfection selon la revendication 1 ou la revendication 2, dans lequel les moyens d'émission comprennent au moins une lampe (9), disposée dans la cavité (8) recouverte par ladite paroi résistante (5).

4. Dispositif de désinfection selon la revendication 3, dans lequel la lampe (9) émettrice de rayons ultraviolets est une lampe au mercure adaptée pour émettre majoritairement des rayons lumineux ayant une longueur d'ondes voisine de 254 nm.

5. Dispositif de désinfection selon l'une quelconque des revendications 3 et 4, comportant en outre des moyens de réflexion (8) des rayons ultraviolets, disposés sous chaque lampe (9).

6. Dispositif de désinfection selon l'une quelconque des revendications précédentes, dans lequel les moyens de commande (15-20) comprennent, d'une part, des moyens de déclenchement (15) pour faire débuter l'émission des rayonnements germicides, et d'autre part, des moyens de temporisation (16, 17) pour faire fonctionner les moyens d'émission (9) pendant une durée prédéterminée à partir de l'actionnement des moyens de déclenchement.

7. Dispositif de désinfection selon la revendication 6, dans lequel la durée prédéterminée est comprise entre 1 et 10 s.

8. Dispositif de désinfection selon l'une quelconque des revendications précédentes, dans lequel les moyens de commande (15-20) comportent des moyens de sécurité (15) nécessitant une clé d'accès pour faire fonctionner les moyens d'émission (9).

9. Dispositif de désinfection selon l'une quelconque des revendications précédentes, dans lequel les moyens de commande (15-20) comportent au moins un capteur de présence (19) adapté pour vérifier la présence d'un utilisateur posant au moins un pied sur la surface d'appui supérieure (3) du dispositif de désinfection, et pour interdire l'émission des rayons ultraviolets tant que cette présence n'est pas détectée.

10. Dispositif de désinfection selon l'une quelconque des revendications précédentes, comportant en outre des moyens d'occultation (10) adaptés pour opacifier la surface d'appui supérieure (3) au moins autour des chaussures (6) de l'utilisateur, en empêchant l'essentiel des rayons ultraviolets de se propager vers le haut au-delà desdites chaussures.

11. Dispositif de désinfection selon la revendication 10, dans lequel les moyens d'occultation comprennent au moins une paroi souple (10) qui recouvre la surface d'appui supérieure (3) et au moins partiellement les chaussures de l'utilisateur.

## Patentansprüche

1. Vorrichtung zur Desinfektion von Schuhsohlen (7) eines Benutzers, wobei diese Vorrichtung dazu bestimmt ist, im wesentlichen in Bodenhöhe installiert zu werden, und eine obere Stützfläche (3) aufweist, auf die der Benutzer zur Desinfektion seiner Schuhsohlen seine Füße stellen kann, **dadurch gekennzeichnet, dass** die obere Stützfläche (3) eine widerstandsfähige und für Ultraviolettstrahlen durchlässige Wandseite (5) aufweist, die der Aufnahme des Gewichts des Benutzers dient und einen Hohlraum (8) abdeckt, in dem Bestrahlungsmittel (9) angeordnet sind, zur Abgabe von ultravioletten Strahlen In einer für die menschliche Gesundheit akzeptabien Art und Menge, wobei elektrische Steuermittel (15 - 20) für den Betrieb dieser Bestrahlungsmittel (9) vorgesehen sind.

2. Desinfektionsvorrichtung nach Patentanspruch 1, bei der Bestrahlungsmittel (9) zur Abgabe von Ultravioletter Strahlung mit Wellenlängen mindestens in dem Bereich zwischen 200 und 300 nm vorgesehen sind.

3. Desinfektionsvorrichtung nach Patentanspruch 1 oder Patentanspruch 2, bei der die Bestrahlungsmittel mindestens eine Lampe (9) umfassen, die in dem Hohlraum (8) untergebracht ist und welche von der besagten widerstandsfähigen Wandseite (5) abgedeckt ist.

4. Desinfektionsvorrichtung nach Patentanspruch 3, bei welcher die ultraviolette Strahlung abgebenden Lampe (9) eine Quecksilberlampe ist, die dafür vorgesehen ist, hauptsächlich Lichtstrahlen mit einer Wellenlänge von annähernd 254 nm abzugeben.

5. Desinfektionsvorrichtung nach einem der Patentansprüche 3 und 4, die ferner unter jeder Lampe (9) angebrachte Mittel zur Reflexion (8) der ultravioletten Strahlen aufweist.

6. Desinfektionsvorrichtung nach einem der vorstehenden Patentansprüche, bei welcher die Steuermittel (15 - 20) einerseits Auslösemittel (15) für den Beginn der keimtötenden Bestrahlung und andererseits Zeitstellmittel (16, 17) für den Betrieb der Bestrahlungsmittel (9) für eine vorbestimmte Zeitdauer ab Betätigung der Auslösemittel umfassen.

7. Desinfektionsvorrichtung nach Patentanspruch 6, bei der die vorbestimmte Zeitdauer zwischen 1 und 10 Sekunden liegt.

8. Desinfektionsvorrichtung nach einem der vorstehenden Patentansprüche, bei der die Steuermittel (15 - 20) Sicherheitsmittel (15) umfassen, die für den Betrieb der Bestrahlungsmittel (9) einen Zugriffsschlüssel erfordern.

9. Desinfektionsvorrichtung nach einem der vorstehenden Patentansprüche, bei der die Steuermittel (15 - 20) mindestens einen Anwesenheitssensor (19) aufweisen, welcher der Überprüfung auf Anwesenheit eines Benutzers, welche mindestens einen Fuß auf die obere Stützfläche (3) der Desinfektionsvorrichtung setzt, sowie dazu dient, die Abgabe ultravioletter Strahlung zu unterbinden, wenn diese Anwesenheit nicht erfasst wird.

10. Desinfektionsvorrichtung nach einem der vorstehenden Patentansprüche, die ferner Verdunklungsmittel (10) aufweist, die der Verdunklung der oberen Stützfläche (3) mindestens des Bereichs um die Schuhe (6) des Benutzers herum dienen, womit sie verhindern, dass der Hauptanteil der ultravioletten Strahlen sich nach oben über die besagten Schuhe hinaus verbreitet.

11. Desinfektionsvorrichtung nach Patentanspruch 10, bei der die Verdunklungsmittel mindestens eine flexible Wandseite (10) umfassen, welche die obere Stützfläche (3) sowie mindestens teilweise die Schuhe des Benutzers abdeckt.

## Claims

1. Device for disinfecting the soles (7) of a users' shoes, this device being intended to be disposed substantially in the region of the ground and comprising an upper support surface (3) on which the user may place his feet to disinfect the soles of his shoes, **characterised in that** the upper support surface (3) comprises a resistant wall (5) allowing ultraviolet light rays through, this resistant wall being suitable to support the weight of the user and covering a cavity (8) in which are disposed suitable emission means (9) to emit ultraviolet rays of a type and quantity that are acceptable for human health, electrical control means (15 to 20) being provided to operate these emission means (9).

2. Disinfecting device according to claim 1, wherein the emission means (9) are suitable to emit ultraviolet rays having wavelengths included at least in the range from 200 to 300 nm.

3. Disinfecting device according to claim 1 or claim 2, wherein the emission means comprise at least one lamp (9), disposed in the cavity (8) covered by said resistant wall (5).

4. Disinfecting device according to claim 3, wherein the ultraviolet ray-emitting lamp (9) is a mercury lamp suitable for emitting mostly light rays having a neighbouring wavelength of 254 nm.

5. Disinfecting device according to either of claims 3 and 4, also comprising ultraviolet ray-reflecting means (8) disposed under each lamp (9).

6. Disinfecting device according to any of the preceding claims, wherein the control means (15 to 20) comprise, on the one hand, release means (15) to trigger the emission of germicidal radiation and, on the other hand, delaying means (16, 17) to operate the emission means (9) over a predetermined period once the release means have been activated.

7. Disinfecting device according to claim 6, wherein the predetermined period is between one and ten seconds.

8. Disinfecting device according to any of the preceding claims, wherein the control means (15 to 20) comprise safety means (15) requiring an access key in order to operate the emission means (9).

9. Disinfecting device according to any of the preceding claims, wherein the control means (15 to 20) comprise at least one presence-sensing device (19) suitable to check the presence of a user placing at least one foot on the upper support surface (3) of the disinfecting device, and to prevent the emission of ultraviolet rays where this presence is not detected.

10. Disinfecting device according to any of the preceding claims, also comprising screening means (10) suitable to make the upper support surface (3) opaque at least around the user's shoes (6) by preventing the majority of the ultraviolet rays from spreading upwards above said shoes.

11. Disinfecting device according to claim 10 wherein the screening means comprise at least one soft wall (10) which covers the upper support surface (3) and at least partially the shoes of the user.
